# EUROPEAN PATENT SPECIFICATION

(11) **EP 3 539 485 B1**
(45) Date of publication and mention of the grant of the patent: **14.02.2024**
(21) Application number: 19160441.2
(22) Date of filing: 04.03.2019
(51) Int. Cl.: A61B 17/221

(54) **SMALL FRAGMENT RETRIEVAL DEVICE**
VORRICHTUNG ZUR WIEDERAUFFINDUNG KLEINER FRAGMENTE
DISPOSITIF D'EXTRACTION DE FRAGMENTS DE PETITE TAILLE

(30) Priority: 15.03.2018 US 201815922027
(43) Date of publication of application: 18.09.2019
(73) Proprietor: Gyrus ACMI, Inc. d/b/a/ Olympus Surgical Technologies America, Westborough, MA 01581 (US)
(72) Inventor: Shelton, Kurt, Bedford, MA 01730 (US)
(74) Representative: Noack, Andreas

(56) References cited:
- WO-A1-2016/148747
- US-A- 5 370 653
- US-A1- 2006 293 612
- US-A1- 2007 198 028
- US-A1- 2008 045 881
- US-A1- 2008 275 483

## Description

### FIELD

The present disclosure relates to a medical device. More specifically, the present disclosure relates to a device for retrieving small fragments.

### BACKGROUND

The statements in this section merely provide background information related to the present disclosure and may or may not constitute prior art.

Lithotripsy is a common method for fragmenting stones, or calculi, in the urinary tract, kidneys, and bladder. Lithotripsy, however, can leave stone fragments, which act as nucleation sites for future stone formation, for example, in the lower pole of a kidney. These fragments remain in the patient after the procedure mostly because of the difficulty in capturing very small fragments of dust employing conventional removal devices such as, for example, stone baskets.

Typical corrective actions may include one or more of the following: monitoring for future stone formation, performing additional ureteroscopic procedures, or percutaneous nephrolithotomy. Among the literature that can pertain to this technology include the following patent documents and published patent applications: EP 2355717, US 20090136594, JP 2006314811 , US 7,883,516, and US 8,753,351.

Prior art patent WO 2016/148747 A1 discloses a small fragment retrieval device, forming the basis for the preamble of claim 1.

### SUMMARY

Accordingly, there is a need for a device that can be employed with lithotripsy that removes small fragments and prevent tissue damage while the device is being pressed against the tissue. It would be desirable to have mechanisms which attract, retain, and contain stone fragments to reliably remove small stone fragments and debris.

The present disclosure provides an improved small fragment retrieval device and a non-claimed method of using the small fragment retrieval device.

According to certain embodiments, a medical device for retrieving small fragments from within a patient's anatomy is provided. The medical device includes a plurality of tips disposed at a distal end of a collection wire and configured to capture stone fragments within the patient's anatomy. Further, the medical device includes an atraumatic tip positioned at the distal end of the collection wire and configured to avoid damage to the patient's anatomy at times the collection wire is manipulated by a user. The main embodiment of the invention is defined in claim 1. Preferred embodiments are described in the dependent claims.

According to exemplary embodiments, not forming part of the claimed invention, a method of collecting stone fragments from a patient's anatomy is provided. The method may include inserting into the patient's anatomy a collection wire having an atraumatic tip positioned at a distal end and configured to avoid damage to an anatomical region at times the collection wire is manipulated by a user. The method may further include positioning the distal end of the collection wire near the anatomical region containing stone fragments. The distal end may include a plurality of tips. Further, the method may include manipulating the collection wire to capture the stone fragments to a plurality of tips at the distal end of the collection wire.

### DRAWINGS

The forgoing and other features and advantages provided by the present disclosure may be better understood by referring to the following description taken in conjunction with the accompanying drawings. The drawings are not necessarily to scale, emphasis instead generally being placed upon illustrating the principles of the technology.
FIG. 1A illustrates a side view of a small fragment retrieval device according to certain embodiments.
FIG. 1B illustrates a side view of the small fragment retrieval device shown in FIG. 1A according to certain embodiments.
FIG. 2 illustrates a side view of another small fragment retrieval device according to certain embodiments.
FIGs. 3A and 3B illustrate side views of a small fragment retrieval device according to certain embodiments.
FIG. 4 illustrate a side view of a portion of the retrieval device shown in FIGs. 3A and 3B coated with an attractant according to certain embodiments.
FIG. 5A illustrates a side view of a small fragment retrieval device in according to the invention.
FIG. 5B illustrates a side view of the small fragment retrieval device shown in FIG. 5A according to the invention.

### DETAILED DESCRIPTION

Existing technologies describe the delivery of object removal devices through catheters. These object removal devices are described as attached to delivery devices such as a guidewire or a catheter. Existing devices primarily target removal of objects from blood vessels such as clots and extend them to apply to removing renal calculi. However, many of these devices suffer from the same limitations of the necessity to atraumatically remove often hard, sharp fragments of calculi from narrow, fragile tubes in the body such as the ureter. Physicians will often utilize the delivery of irrigation fluid to encourage small calculi fragments in the ureter to pass into the bladder where they will spontaneously pass from the patient with normal urine passage.

However, there remains a challenge with removing small calculi fragments that inter the kidney during lithotripsy. In this case, the complex geometry of the kidney creates turbulence patterns that are more difficult to predict making it more challenging to remove small calculi fragments by irrigation. Using small fragment removal devices creates risks of perforations and other injury to the kidney since the devices are being delivered into a closed structure not an open tube such as a vein or artery. A steerable guidewire tip may not be sufficient to prevent trauma, and it also may act to maintain a separation between the fragment capture elements and the distal most end of the fragment removal device where the fragments are often located. Example embodiments of the present invention address the need for a fragment removal device capable of atraumatically retrieving small fragments from the body even in closed passages in the body such as renal calyces.

The following description is merely exemplary in nature and is not intended to limit the present disclosure, application, or uses. The scope of the invention is defined by the appended claims.

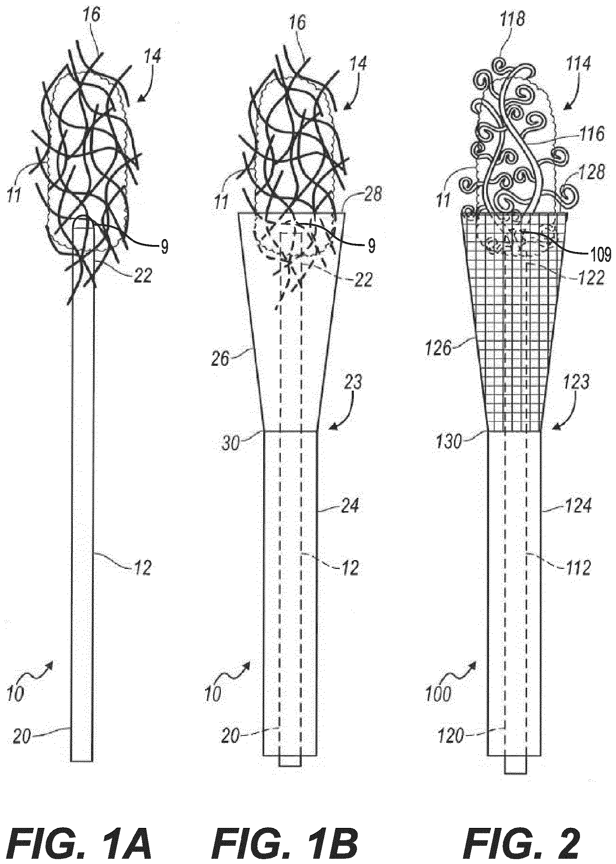

Referring now to the drawings, a small fragment retrieval device or collection wire embodying the principles of the present disclosure is illustrated in FIG. 1A and designated at 10. The collection wire 10 includes a shaft 12 with a proximal end 20 and a distal end 22. A plurality of tips 16 is attached to the distal end 22 of the shaft 12. In the configuration shown in FIG. 1A, the plurality of tips 16 is a plurality of filaments that are intertwined into a fibrous structure 14. The fibrous structure 14 is porous to allow fluid to flow through it while capturing small stone fragments and dust particles that are generated during lithotripsy. An atraumatic tip 9 is positioned at the distal end 22 of the collection wire 10.

In one embodiment, the tips of the plurality of tips 16 may extend outwardly from the distal end 22 of the collection wire 10. In one embodiment, the tips of the plurality of tips may extend radially from the collection wire 10. In one embodiment, the tips of the plurality of tips may extend laterally from the collection wire 10. In one embodiment, the plurality of tips 16 may extend radially from the collection wire 10. In some embodiments, the plurality of tips 16 may extend outwardly from the collection wire 10. In some embodiments, the tips of the plurality of tips 16 being spaced apart and positioned radially about the distal end 22 of the collection wire 10.

When in use, a user, e.g., a physician, inserts the collection wire 10 into a patient's anatomy so that the fibrous structure 14 is positioned near an anatomical region of the patient that contains small stone fragments and dust which may have been produced, for example, by lithotripsy. As the physician sweeps the fibrous structure 14 around the anatomical region, the small stone fragments and dust are collected by the filaments 16. After the stone fragments and dust have been accumulated into a conglomeration of stone fragments 11, the physician pulls on the shaft 12 to retrieve the collection wire 10 along with the conglomeration of stone fragments 11 from the patient's anatomy.

As stated above, the atraumatic tip 9 is positioned at the distal end 22 of the collection wire 10. The atraumatic tip 9 is configured to be axially pressed into the surface of a tissue, e.g., renal pelvis, without causing damage to the tissue thereby permitting the fibrous structure 14 or the collection wires 16 to engage with the stone fragments.

The atraumatic tip 9 may be any shape capable of dispersing over a larger tissue surface area when an axial pressure is delivered by the proximal end of a guide wire or catheter as the collection wire 10 is being moved through the fragments to be removed. The atraumatic tip 9 may be spherical, coiled, helically coiled, half spherical, oval, cylindrical, flexibly conical, or any shape without substantially sharp edges or corners. The shape of the atraumatic tip 9 can be affected by the materials being used to fabricate the atraumatic tip. The atraumatic tip 9 can be made with harder materials, e.g., materials with Shore A = 30 to 100 or Shore D = 0 to 50 modulus, or with softer materials, e.g., Shore A = 0 to 20 or Shore 00 = 1 to 70 modulus. The atraumatic tip 9 shapes can be selected from those that provide a tissue interaction area that creates pressures of less than 0.02 Pascal when axial forces in the approximate range of 50 to 500g are applied to the delivery guidewire or catheter. The atraumatic tip 9 can be made into a wider range of shapes if softer materials are used (e.g., Shore 0 to 20) as they will deform and have a higher effective surface area to distribute the high axial forces over.

The atraumatic tip 9 can be made from biocompatible metals, e.g., NiTi (nickel titanium), Ti (titanium), gold, or stainless steel. The atraumatic tip 9 can also be made from biocompatible polymers, e.g., polyurethane, polyethylene copolymers, ethelenevinylacetate, acrylates, and their gels such as polyacrylamide gel and polyacrylic acids.

The fibrous structure 14 may include synthetic balls of material comprising rayon or polyester, ultra-high-molecular-weight polyethylene (UHMWPE), other polymers, or the like. Individual filaments of the fibrous structure may be coated with a flexible polymer or adhesive, such as for example amyloid fiber, natural amyloid fiber, mussel foot proteins (Mfps), or bundles of carbon nanotubes to make use of microscale van der Waals forces to capture the small stone fragments or dust (that is, "gecko tape").

Referring to FIG. 1B, the collection wire 10 may include an optional containment device 23. The containment device 23 includes a sheath 24 and an expandable cone-shaped basket 26 attached to a distal end 30 of the sheath 24. The cone-shape basket 26 may be made from a solid material or from a mesh.

When in use, the sheath 24 and the basket 26 are initially placed over the shaft 12 of the collection wire 10 such that basket 26 is collapsed about the shaft 12. After the conglomeration of stone fragments 11 is collected by the filaments 16 as described above, the physician pulls the proximal end 20 of the shaft 12 relative to the containment device 23. As this occurs, the basket expands or opens as the conglomeration of stone fragments 11 is pulled into the basket 26. The physician then removes the collection wire 10 along with the conglomeration of stone fragments 11 contained in the basket 26 from the patient's anatomy.

Turning now to FIG. 2, there is shown an alternative small fragment retrieval device or collection wire 100 that includes a shaft 112 with a proximal end 120 and a distal end 122. A plurality of tips 114 is attached to the distal end 122 of the shaft 112. The plurality of tips 114 includes a plurality of primary curled tips 116 and a plurality of secondary curled tips 118 attached to the primary curled tips 116. The collection wire 100 may include a containment device 123 that includes a sheath 124 and an expandable basket 126 attached to a distal end 130 of the sheath 124. The basket 126 may be formed from a solid material or may have a mesh structure.

When in use, the sheath 124 and the basket 126 are initially placed over the shaft 112 such that the basket 126 is collapsed about the shaft 112. The collection wire 100 is inserted into a patient's anatomy so that the plurality of tips 114 is positioned near an anatomical region of the patient that contains small stone fragments and dust. As a physician sweeps the plurality of tips 114 around the anatomical region, the small stone fragments and dust are collected by the primary and secondary curled tips 116 and 118. After the stone fragments and dust have been accumulated into the conglomeration of stone fragments 11, the physician pulls on the proximal end 120 of the shaft 112 so that the conglomeration of stone fragments is pulled into the basket 126 as the basket 126 expands to an open state. The physician then retrieves the collection wire 100 along with the conglomeration of stone fragments 11 contained in the basket 126 from the patient's anatomy. In certain procedures, the collection wire 100 can be employed without the use of the containment device 123.

The plurality of tips 14 and 114 described above are generally flexible and are made of any suitable material such as, for example, plastic, silicone or metal. The filaments 16 of the collection wire 10 and the primary and secondary curled tips 116 and 118 of the collection wire 100 can be coated with an adhesive material. For example, the adhesive may be a calcium attractant adhesive. It is contemplated that the filaments, collection wire, and/or curled tips may be coated (for example, sinter bonded) with calcium oxalate or an alternative calcium-based mineral. Subsequently, a nanofunctional molecule, which may be a protein or a polyanionic macromolecule and which may be provided with a calcium attractive surface at both ends, may be applied to the filaments, collection wire, and/or curled tips so as to capture stone dust or stone debris containing calcium oxalate. The nanofunctional molecule could be carboxylic acid-rich proteins, osteopontin, or prothrombin fragment 1, for example, or the like, and may be crosslinked with a second nanofunctional molecule. In this way, the nanofunctional molecule may be calcium attractive at two opposing ends and thereby attract and retain stone dust and debris to the filaments, curled tips, and/or collection wire. The nanofunctional molecule may be long enough such that multiple calcium attractive sites are provided on a single molecule. In this case, the nanofunctional molecule would be sufficient to attract and retain stone dust and debris to the filaments, curled tips, and/or collection wire.

Alternatively, the filaments 16 and the primary and secondary curled tips 116 and 118 may be coated with magnetic material to act as a magnetic attractant. Contacting kidney stones with ferrous or magnetic particles that are able to bind the kidney stone debris, and reacting the kidney stone debris with the ferrous or magnetic particles may cause the kidney stone debris to become attractable magnetically. In one aspect, the ferrous or magnetic particles may further comprise an agent that specifically binds the surface of the kidney stone debris selected from proteins that interact with calcium-based biominerals, such as carboxylic acid-rich proteins, osteopontin, prothrombin fragment 1. The magnetic protein ferritin may be used as a second protein and cross-linked to one of the nanofunctional proteins, or first proteins, mentioned above and used for this purpose.

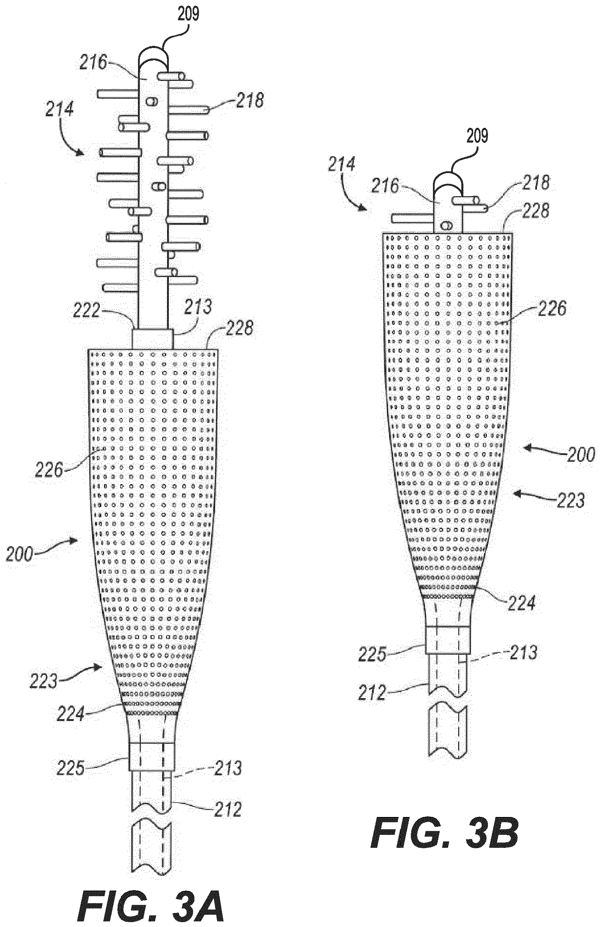

Referring now to FIGs. 3A and 3B, there is shown yet another small fragment retrieval device or collection wire 200. The collection wire 200 includes a shaft 213 and a plurality of tips 214. The plurality of tips 214 may be formed integrally with a distal portion of the shaft 213, or the plurality of tips 214 may include a primary projection 216 attached the distal end 222 of the shaft 213. The plurality of tips 214 further includes a collection of projections 218 that extend substantially laterally from the distal portion of the shaft 213 or from the primary projection 216. As illustrated in FIG. 3A, the projections 218 are spaced apart and are arranged in a spiral pattern or arranged about the primary projection 216. The plurality of tips can be coated with an adhesive, such as, for example, calcium attractant adhesive or a magnetic attractant.

The collection wire 200 also includes a containment device 223. The containment device 223 includes a sheath 212 and a basket 224 attached to a distal end 225 of the sheath 212. The basket 224 may be made of a solid material or a mesh-like structure with a plurality of holes 226.

When in use, the sheath 212 and the basket 224 are placed over the shaft 213. A physician inserts the retrieval device 200 into a patient's anatomy so that the plurality of tips 214 is positioned near an anatomical region of the patient that contains small stone fragments and dust. As the physician sweeps the plurality of tips 214 around the anatomical region, the small stone fragments and dust are collected by the collection of projections 218. After the stone fragments have been accumulated into a conglomeration of stone fragments, the physician pulls on the proximal end of the shaft 212 so that the conglomeration of stone fragments and dust are pulled into a distal end 228 of the basket 226. The physician then retrieves the collection wire 200 along with the conglomeration of stone fragments and dust contained in the basket 224 from the patient's anatomy. In certain procedures, the collection wire 200 can be employed without the use of the containment device 223.

Various substances and exemplary methods not forming part of the invention, may be used to produce a flocculation or aggregation of stone dust or debris particulates prior to the various collection exemplary methods described herein being commenced. For example, flocculation may be accomplished through the creation of a low pH environment in the kidney. Further, adding, ejecting, and/or inserting an adhesive agent such as polyethylenimine, biocompatible nanoadhesive such as alginate gel, cellulose gel, protein based gel, collagen, polysaccharide, chitosan gel, in vivo gel formulations which at least in part polymerize and/or gel at the target site in a human body to form a biocompatible hydrogel polymer, ferrogels, and/or biocompatible magnetic gels which may include polyvinyl alcohol and gluteraldehyde may help to aggregate stone dust or stone debris. Such an adhesive agent may be added, injected, and/or inserted with an additional device such as a catheter or out of an opening in a distal most end of the primary projection 216, for example. The adhesive agent may be given some time to collect stone dust or stone debris, the primary projection 216 or other collection device may be inserted into the region containing the adhesive agent to attract the adhesive agent containing stone dust and stone debris, the containment device 223 may surround the primary projection 216 or other collection device, and the device may be subsequently removed from a patient.

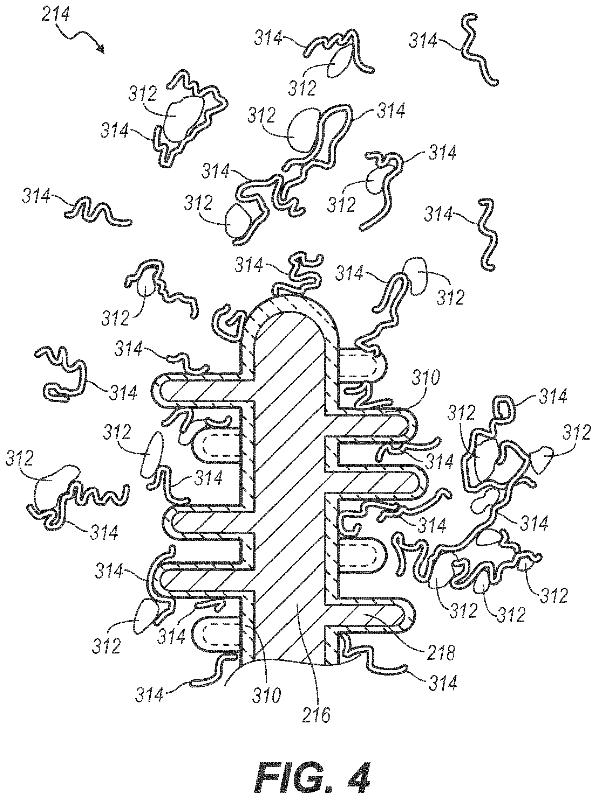

In some arrangements, the plurality of tips 214, such as, for example, the primary projection 216 and the collection of projections 218, may be coated with a calcium attractant adhesive, similar to the coating described previously with respect to filaments 16 of the collection wire 10 and the primary and secondary curled tips 116 and 118 of the collection wire 100. For example, the plurality of tips 214 may be coated (for example, sinter bonded) with calcium oxalate or an alternative calcium-based mineral 310, as shown in FIG. 4. Subsequently, a nanofunctional molecule 314, which may be a protein or a polyanionic macromolecule and which may be provided with a calcium attractive surface at both ends, is applied to the plurality of tips 214 so as to attract stone dust or stone debris 312 containing calcium oxalate. The nanofunctional molecule 314 could be carboxylic acid-rich proteins, osteopontin, or prothrombin fragment 1, for example, or the like, and may be crosslinked with a second nanofunctional molecule. In this way, the nanofunctional molecule 314 may be calcium attractive at two opposing ends and thereby attract and retain stone dust and debris to the plurality of tips 214. Further, the nanofunctional molecule 314 may be long enough such that multiple calcium attractive sites are provided on a single molecule. In this case, the nanofunctional molecule would be sufficient to attract and retain stone dust and debris 312 to the plurality of tips 214.

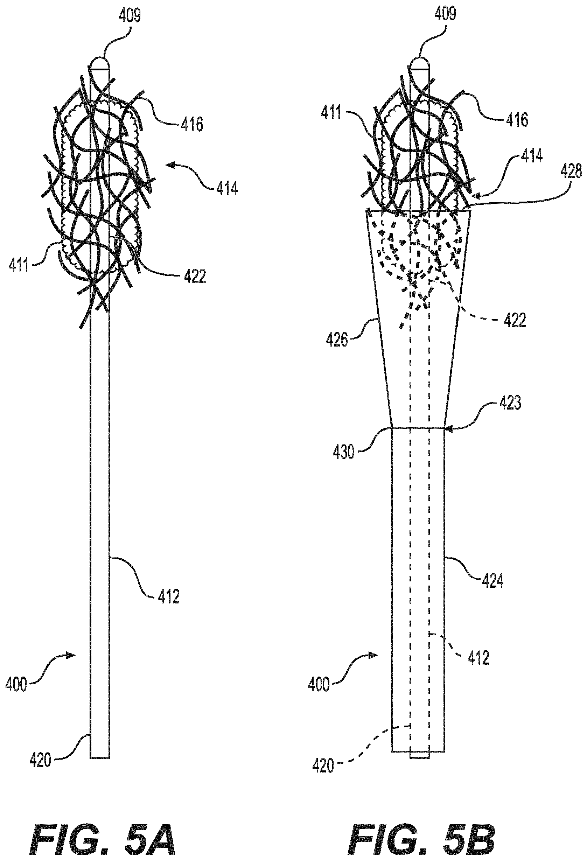

FIGs. 5A and 5B illustrate the main embodiment of the small fragment retrieval device, as described in claim 1. As shown, the collection wire 400 includes a shaft 412 having a distal end 422 and proximal end 420. A plurality of tips 416 is attached to the distal end 422 of the shaft 412. The plurality of tips 416 can be a plurality of filaments that are intertwined into a fibrous structure 414. As stated above, the fibrous structure 414 is porous to allow fluid to flow through it while capturing small stone fragments and dust particles that are generated during lithotripsy.

Further, the collection wire 400 includes an atraumatic tip 409 positioned at the distal end 422 of the collection wire 400 and not within the fibrous structure 414. The atraumatic tip 409 is configured for being axially pressed into the surface of a tissue, e.g., renal pelvis, without causing damage to the tissue thereby permitting fibrous structure 414 or collection wires 416 to engage with the stone fragments. Although, the atraumatic tip 409 is not placed within the fibrous structure 414, it will not cause damage to the tissue as the atraumatic tip 409 is capable of dispersing over a larger tissue surface area at times axial pressure being delivered to the collection wire 400.

Referring further to FIG. 5B, the collection wire 400 may include an optional containment device 423. The containment device 423 includes a sheath 424 and an expandable cone-shaped basket 426 attached to a distal end 430 of the sheath 424. The cone-shape basket 426 may be made from a solid material or from a mesh.

When in use, the sheath 424 and the basket 426 are initially placed over the shaft 412 of the collection wire 400 such that basket 426 is collapsed about the shaft 412. After the conglomeration of stone fragments 411 is collected by the filaments 16 as described above, the physician pulls the proximal end 420 of the shaft 412 relative to the containment device 423. As this occurs, the basket expands or opens as the conglomeration of stone fragments 411 is pulled into the basket 426. The physician then removes the collection wire 400 along with the conglomeration of stone fragments 411 contained in the basket 426 from the patient's anatomy.

The description of the invention is merely exemplary in nature and variations that do not depart from the scope of the appended claims are intended to be within the scope of the invention.

## Claims

1. A medical device configured for entering a patient's anatomy comprising:
a plurality of tips (416) disposed at a distal end (422) of a collection wire (400) and configured to capture stone fragments within the patient's anatomy,
the plurality of tips (416) comprise a plurality of filaments intertwined into a fibrous structure (414); and
an atraumatic tip (409) positioned at the distal end of the collection wire (400), and configured to avoid damage to the patient's anatomy at times the collection wire (400) is manipulated by a user,
**characterized in that** the atraumatic tip (409) extends outside of the fibrous structure (414).

2. The medical device of claim 1, wherein the tips of the plurality of tips (416) comprise a plurality of curled tips (116, 118).

3. The medical device of claim 1, wherein the plurality of filaments are coated with a flexible polymer or an adhesive.

4. The medical device of any of claims 1 to 3, further comprising a wire mesh (426) that selectively surrounds the fibrous structure (414).

5. The medical device of claim 4, wherein the wire mesh (426) is configured to move distally and proximally relative to the fibrous structure (414).

6. The medical device of claim 5, wherein the wire mesh (426) is configured to collapse over the fibrous structure (414).

7. The medical device of any claims 1 to 6, wherein the collection wire (400) is formed of molded silicone.

8. The medical device of any of claims 1 to 6, wherein the collection wire (400) is formed of molded plastic.

9. The medical device of any of claims 1 to 8, wherein the plurality of tips (416) includes at least one of:
i) a magnetic outer surface; and
ii) a nanofunctional molecule bonded to an outer surface.

10. The medical device of any of claims 1 to 9, wherein the atraumatic tip (409) is configured to disperse a force over a surface of the patient's anatomy.

11. The medical device of claim 10, wherein the atraumatic tip (409) is in a form of a sphere, half sphere, cylinder, coil, oval, semi-round object, or cone.

12. The medical device of any of claims 1 to 11, wherein the atraumatic tip (409) is made of any material having a hardness of 0 to 100 Shore.

13. The medical device of any of claims 1 to 12, wherein the atraumatic tip (409) is made of a biocompatible metal, a biocompatible polymer, or both.

## Patentansprüche

1. Eine medizinische Vorrichtung, die dazu eingerichtet ist, in die Anatomie eines Patienten einzudringen, umfassend:
eine Vielzahl von Spitzen (416), die an einem distalen Ende (422) eines Sammeldrahtes (400) angeordnet und dazu eingerichtet sind, Steinfragmente innerhalb der Anatomie des Patienten aufzunehmen,
die Vielzahl von Spitzen (416) umfassen eine Vielzahl von Filamenten, die zu einer faserigen Struktur (414) verflochten sind; und
eine atraumatische Spitze (409), die am distalen Ende des Sammeldrahtes (400) positioniert ist und dazu eingerichtet ist, eine Beschädigung der Anatomie des Patienten zu vermeiden, wenn der Sammeldraht (400) von einem Benutzer gehandhabt wird,
**dadurch gekennzeichnet, dass** sich die atraumatische Spitze (409) außerhalb der faserigen Struktur (414) erstreckt.

2. Die medizinische Vorrichtung nach Anspruch 1, wobei die Spitzen der Vielzahl von Spitzen (416) eine Vielzahl von gekräuselten Spitzen (116, 118) umfassen.

3. Die medizinische Vorrichtung nach Anspruch 1, wobei die Vielzahl von Filamenten mit einem flexiblen Polymer oder einem Klebstoff beschichtet ist.

4. Die medizinische Vorrichtung nach einem der Ansprüche 1 bis 3, weiterhin umfassend ein Drahtgeflecht (426), das die faserige Struktur (414) selektiv umgibt.

5. Die medizinische Vorrichtung nach Anspruch 4, wobei das Drahtgeflecht (426) dazu eingerichtet ist, sich distal und proximal relativ zu der faserigen Struktur (414) zu bewegen.

6. Die medizinische Vorrichtung nach Anspruch 5, wobei das Drahtgeflecht (426) dazu eingerichtet ist, sich über die faserige Struktur (414) zusammenzuziehen.

7. Die medizinische Vorrichtung nach einem der Ansprüche 1 bis 6, wobei der Sammeldraht (400) aus gegossenem Silikon gebildet ist.

8. Die medizinische Vorrichtung nach einem der Ansprüche 1 bis 6, wobei der Sammeldraht (400) aus gegossenem Kunststoff gebildet ist.

9. Die medizinische Vorrichtung nach einem der Ansprüche 1 bis 8, wobei die Mehrzahl von Spitzen (416) mindestens eines der folgenden Merkmale aufweist:
i) eine magnetische äußere Oberfläche; und
ii) ein nanofunktionelles Molekül, das an eine äußere Oberfläche gebunden ist.

10. Die medizinische Vorrichtung nach einem der Ansprüche 1 bis 9, wobei die atraumatische Spitze (409) dazu eingerichtet ist, eine Kraft über eine Oberfläche der Anatomie des Patienten zu verteilen.

11. Die medizinische Vorrichtung nach Anspruch 10, wobei die atraumatische Spitze (409) die Form einer Kugel, Halbkugel, eines Zylinders, einer Spirale, eines Ovals, eines halbrunden Objekts oder eines Kegels hat.

12. Die medizinische Vorrichtung nach einem der Ansprüche 1 bis 11, wobei die atraumatische Spitze (409) aus einem beliebigen Material mit einer Härte von 0 bis 100 Shore hergestellt ist.

13. Die medizinische Vorrichtung nach einem der Ansprüche 1 bis 12, wobei die atraumatische Spitze (409) aus einem biokompatiblen Metall, einem biokompatiblen Polymer oder beidem hergestellt ist.

## Revendications

1. Un dispositif médical configuré pour entrer dans l'anatomie d'un patient comprenant :
une pluralité de pointes (416) disposées à l'extrémité distale (422) d'un fil de collecte (400) et configurées pour capturer des fragments de pierre dans l'anatomie du patient,
la pluralité de pointes (416) comprend une pluralité de filaments entrelacés dans une structure fibreuse (414) ; et
une pointe atraumatique (409) positionnée à l'extrémité distale du fil de collecte (400) et configurée pour éviter d'endommager l'anatomie du patient lorsque le fil de collecte (400) est manipulé par un utilisateur,
**caractérisé en ce que** la pointe atraumatique (409) s'étend à l'extérieur de la structure fibreuse (414).

2. Le dispositif médical de la revendication 1, dans lequel les pointes de la pluralité de pointes (416) comprennent une pluralité de pointes enroulées (116, 118).

3. Le dispositif médical de la revendication 1, dans lequel la pluralité de filaments est recouverte d'un polymère flexible ou d'un adhésif.

4. Le dispositif médical de l'une des revendications 1 à 3, comprenant en outre un treillis (426) qui entoure sélectivement la structure fibreuse (414).

5. Le dispositif médical de la revendication 4, dans lequel le treillis (426) est configuré pour se déplacer distalement et proximalement par rapport à la structure fibreuse (414).

6. Le dispositif médical de la revendication 5, dans lequel le treillis (426) est configuré pour se replier sur la structure fibreuse (414).

7. Le dispositif médical de l'une des revendications 1 à 6, dans lequel le fil de collecte (400) est formé de silicone moulé.

8. Le dispositif médical de l'une des revendications 1 à 6, dans lequel le fil de collecte (400) est formé de plastique moulé.

9. Le dispositif médical de l'une des revendications 1 à 8, dans lequel la pluralité de pointes (416) comprend au moins l'un des éléments suivants :
i) une surface extérieure magnétique ; et
ii) une molécule nanofonctionnelle liée à une surface extérieure.

10. Le dispositif médical de l'une des revendications 1 à 9, dans lequel la pointe atraumatique (409) est configurée pour disperser une force sur une surface de l'anatomie du patient.

11. Le dispositif médical de la revendication 10, dans lequel la pointe atraumatique (409) se présente sous la forme d'une sphère, d'une demisphère, d'un cylindre, d'une bobine, d'un ovale, d'un objet semi-rond ou d'un cône.

12. Le dispositif médical de l'une des revendications 1 à 11, dans lequel la pointe atraumatique (409) est constituée d'un matériau ayant une dureté de 0 à 100 Shore.

13. Le dispositif médical de l'une des revendications 1 à 12, dans lequel la pointe atraumatique (409) est constituée d'un métal biocompatible, d'un polymère biocompatible ou des deux.
